(19)

Europäisches Patentamt
European Patent Office
Office européen des brevets

(11)  **EP 4 432 292 A1**

(12)  **EUROPEAN PATENT APPLICATION**
published in accordance with Art. 153(4) EPC

(43) Date of publication:
**18.09.2024  Bulletin 2024/38**

(21) Application number: **22871088.5**

(22) Date of filing: **01.07.2022**

(51) International Patent Classification (IPC):
**G16C 20/10** (2019.01)      **G16C 10/00** (2019.01)

(86) International application number:
**PCT/CN2022/103299**

(87) International publication number:
**WO 2023/077843 (11.05.2023 Gazette 2023/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **08.11.2021   CN 202111323701**

(71) Applicant: **Nanjing Institute of Microinterface Technology Co., Ltd**
**Nanjing, Jiangsu 210047 (CN)**

(72) Inventors:
• **ZHANG, Zhibing**
  **Nanjing, Jiangsu 210047 (CN)**
• **ZHOU, Zheng**
  **Nanjing, Jiangsu 210047 (CN)**

• **ZHANG, Feng**
  **Nanjing, Jiangsu 210047 (CN)**
• **LI, Lei**
  **Nanjing, Jiangsu 210047 (CN)**
• **TIAN, Hongzhou**
  **Nanjing, Jiangsu 210047 (CN)**
• **WEI, Gelin**
  **Nanjing, Jiangsu 210047 (CN)**
• **LU, Wenjun**
  **Nanjing, Jiangsu 210047 (CN)**
• **LI, Xiabing**
  **Nanjing, Jiangsu 210047 (CN)**
• **LI, Yongqiang**
  **Nanjing, Jiangsu 210047 (CN)**

(74) Representative: **Zaboliene, Reda et al**
**Metida**
**Business center Vertas**
**Gyneju str. 16**
**01109 Vilnius (LT)**

(54)  **METHOD FOR EVALUATING ENHANCEMENT DEGREE OF MICRO-INTERFACE REACTION FOR PREPARING BUTYRALDEHYDE BY MEANS OF HYDROFORMYLATION OF PROPYLENE**

(57)    The invention provides an evaluation method for a strengthening degree of a micro-interface reaction of propylene hydro-formylation to produce butyraldehyde, comprising the following steps: (A) establishing an expression of a reaction rate of propylene hydro-formylation; (B) constructing a macroscopic reaction kinetics equation of a reaction gas; (C) establishing a mathematical expression of a reaction enhancement factor; (D) calculating a concentration of each reaction component in a liquid phase. The invention aims at realizing the economy of the micro-interface reaction strengthening technology, and improves the space-time yield to the greatest extent by determining the evaluation method of the oxidation reaction micro-interface reaction strengthening, which is beneficial to optimize the design and operation of the butyraldehyde synthesis reactor.

Fig. 1

**Description**

**FIELD OF THE INVENTION**

The present invention relates to the technical field of reactors and modeling, in particular to an evaluation method for a strengthening degree of a micro-interface reaction of propylene hydro-formylation to produce butyraldehyde.

**BACKGROUND OF THE INVENTION**

The oxo reactor is the core equipment in the whole oxo process, and the Davy-Dow process uses two stirred tank reactors to react in series. The raw materials of propylene hydro-formylation reaction are propylene and synthesis gas, the catalyst is rhodium phosphine complex of ligand triphenylphosphine, and the main reaction products are n-butyraldehyde and isobutyraldehyde. The synthesis gas enters the reactor through the gas distributor, and the propylene enters the reactor through the external circulation pipeline. The synthesis gas is broken into bubbles under the action of the stirring paddle, and the two phases (including gas and liquid) are mixed, mass-transferred and reacted. The reaction temperature is 95 ~ 110°C, the reaction pressure is 1.8 ~ 1.9 MPa, the conversion rate of propylene is about 95%, the yield rate of butyraldehyde is about 95%, and the utilization rate of the synthesis gas is more than 97%. In addition to the main reaction, some side reactions also occur, such as the hydrogenation of propylene to propane, the further hydrogenation of butyraldehyde to butanol, and the condensation of aldehydes to form trimers and high boilers.

From the perspective of macroscopic reaction kinetics, the main reasons why original devices adopt higher operating temperatures and higher operating pressures to achieve the production capacity target are: (1) increasing the temperature can increase the intrinsic reaction rate and reduce the reaction resistance; (2) increasing the pressure can increase the partial pressure of reaction gas (CO and $H_2$) and increase mass transfer driving force. The macroscopic reaction rate of the system is accelerated due to the reduction of reaction resistance and the increase of mass transfer driving force.

Since high temperature and high pressure conditions will have some adverse effects on actual production, the operation of increasing temperature and increasing pressure is not an ideal way to increase the macroscopic reaction rate. Based on the characteristics of the rhodium-catalyzed hydro-formylation of propylene to butyraldehyde, the process is considered to be a reaction process affected by both mass transfer and intrinsic reaction. Based on the macroscopic reaction kinetic theory, the factors that determine the macroscopic reaction rate include: (1) intrinsic reaction rate; (2) gas partial pressure; (3) mass transfer rate. There are researches mostly focused on how the first two factors affect the macroscopic reaction rate, but there is little research on how to enhance the mass transfer rate.

Theoretical research and industrial practice have shown that micro-interface strengthening technology can greatly increase the gas-liquid interface area and mass transfer rate of multiphase reaction systems. In theory, it can also be proved that the micro-interface strengthening technology is an advanced technology to realize the carbonyl synthesis of propylene, which not only increases production capacity but also reduces energy consumption and material consumption.

A bubble scale structure-effect control model modeling method of a micro-interface strengthening reactor (Publication No.: CN107563051A) quantifies the relationship between the reactor bubble scale and the structural parameters, operating parameters and physical parameters of the reactor. When the ventilation rate is constant, the smaller the bubble scale, the larger the gas-liquid interface area, the smaller the mass transfer resistance, and the larger the macroscopic reaction rate. Therefore, when the production capacity is constant, how to determine the evaluation method of micro-interface reaction strengthening of oxidation reaction, so as to maximize the space-time yield rate for realizing the economics of micro-interface reaction strengthening technology is an important practical problem.

In view of this, the present invention is proposed.

**SUMMARY OF THE INVENTION**

The objective of the present invention is to provide an evaluation method for a strengthening degree of a micro-interface reaction of propylene hydro-formylation to produce butyraldehyde. The method aims to realize the economy of the micro-interface reaction strengthening technology, which maximizes the space-time yield rate by determining the evaluation method of the oxidation reaction micro-interface reaction strengthening, and then optimizes the design and operation of the butyraldehyde synthesis reactor.

The micro-interface strengthening technology can greatly increase the gas-liquid phase interface area and mass transfer rate of the multiphase reaction system. In theory, it can also be proved that the micro-interface strengthening technology is an advanced technology to realize the carbonyl synthesis of propylene, which not only increases production capacity but also reduces energy consumption and material consumption. In fact, according to the macroscopic reaction kinetic theory, the factors that determine the macroscopic reaction rate include: (1) intrinsic reaction rate; (2) gas partial pressure; (3) mass transfer rate.

At present, it's confirmed that the micro-interface strengthening is mainly reflected in two aspects, one is the increase of the gas-liquid mass transfer interface area, and the other is the enhancement of the gas-liquid mass transfer coefficient, that is, the reduction of the gas-liquid mass transfer resistance. In general, when the ventilation rate is constant, the smaller the bubble scale, the larger the gas-liquid interface area, the smaller the mass transfer resistance, and the larger the macroscopic reaction rate. Therefore, when the production capacity is constant, quantifying the strengthening effect of the micro-interface reaction can help to optimize the design and operation of the butyraldehyde synthesis reactor, thereby maximizing the space-time yield rate.

The invention can realize the economy of the micro-interface reaction strengthening technology by determining the evaluation method of the micro-interface strengthening reaction of propylene hydro-formylation to produce butyraldehyde, and simultaneously maximize the space-time yield rate.

In fact, in order to optimize the structure of the micro-interface system, the patent with publication No. CN107563051A quantifies the relationship between the bubble scale of the reactor and the structural parameters, operating parameters and physical parameters of the reactor. This patent reconstructs the research on the algorithm of the system bubble diameter $d_{32}$. Based on the $d_{32}$ constructed by the patent, the present invention constructs the structure-effect control mathematical model of each reaction gas in the reaction system. In order to further explore the evaluation method of the reaction system, the present invention also constructs the reaction equations of each reaction and the mathematical expression of the reaction enhancement factor, etc. The concentration of each reaction component in the liquid phase is calculated simultaneously through these expressions, and it is possible to clearly evaluate the effect of the micro-interface on improving the mass transfer through the calculation results. This evaluation method of the present invention is unique.

In order to realize the above-mentioned objective of the present invention, the following technical schemes are adopted:

The present invention provides an evaluation method for a strengthening degree of a micro-interface reaction of propylene hydro-formylation to produce butyraldehyde, including the following steps:

(A) establishing an expression of a reaction rate of propylene hydro-formylation;

(B) constructing a macroscopic reaction kinetics equation of a reaction gas;

(C) establishing a mathematical expression of a reaction enhancement factor; and

(D) calculating a concentration of each reaction component in a liquid phase.

Preferably, the step (A) includes:

establishing chemical reaction formulas of all reactions in a process of the micro-interface reaction:

$$CH_3CH= CH_2 + CO + H_2 \rightarrow CH_3CH_2CH_2CHO \qquad (1)$$

$$CH_3CH= CH_2 + CO + H_2 \rightarrow CH_3CH(CH_3)CHO \qquad (2)$$

$$CH_3CH= CH_2 + H_2 \rightarrow CH_3CH_2CH_3 \qquad (3)$$

wherein, formula (1) is a main reaction equation, and formulas (2) ~ (3) are side reaction equations;

constructing reaction rate expressions of all reactions according to the chemical reaction formulas:

$$R_{Bn} = \frac{k_B C_{Pe} C_{H_2} C_{CO} \left(1 + k_2 x_{Lg}\right)}{1 + a x_{Lg} + x_{Lg}^{2}} x_{Rh} \qquad (4)$$

$$R_{Bi} = \frac{k_B C_{Pe} C_{H_2} C_{CO}}{1 + a x_{Lg} + b x_{Lg}^{2}} x_{Rh} \qquad (5)$$

$$R_{\mathrm{pe}} = k_{\mathrm{pe}} C_{\mathrm{Pe}} C_{\mathrm{H_2}} x_{\mathrm{Rh}} \qquad (6)$$

wherein, $R_{Bh}$, $R_{Bi}$ and $R_{pe}$ are reaction rates of n-butyraldehyde, isobutyraldehyde and propane, respectively, in mol/(m³·s); $k_B$ and $k_{pe}$ are reaction rate constants of butyraldehyde and propane, respectively, in m⁶/(mol²·s) and m³/(mol·s); $k_2$, $a$ and $b$ are reaction parameters; $C_{\mathrm{Pe}}$, $C_{\mathrm{H_2}}$ and Cco are concentrations of propylene, $H_2$ and CO in the liquid phase, respectively, in mol/m³; $x_{\mathrm{Lg}}$ and $x_{\mathrm{Rh}}$ are mass fractions of ligand and catalyst, respectively.

Preferably, the step (B) includes:

during the reaction process, mass transfer rates of CO and $H_2$ in a gas film and a liquid film are equal to reaction rates in the liquid phase, and the following formulas are obtained:

$$k_{G,\mathrm{CO}} a (P_{\mathrm{CO}}/H_{\mathrm{CO}} - C_{i,\mathrm{CO}}) = k_{L,\mathrm{CO}} a E_{\mathrm{CO}} (C_{i,\mathrm{CO}} - C_{\mathrm{CO}}) = R_{Bn} + R_{Bi} \qquad (7)$$

$$k_{G,\mathrm{H_2}} a (P_{\mathrm{H_2}}/H_{\mathrm{H_2}} - C_{i,\mathrm{H_2}}) = k_{L,\mathrm{H_2}} a E_{\mathrm{H_2}} (C_{i,\mathrm{H_2}} - C_{\mathrm{H_2}}) = R_{Bn} + R_{Bi} + R_{\mathrm{Pe}} \qquad (8)$$

the formula (8) is simplified to obtain a macroscopic reaction rate equation of propylene:

$$-r_{\mathrm{Pe}} = \cfrac{1}{\cfrac{1}{k_{G,\mathrm{H_2}} a} + \cfrac{1}{k_{L,\mathrm{H_2}} a E_{\mathrm{H_2}}} + \cfrac{1}{(R_{Bn} + R_{Bi} + R_{\mathrm{Pe}})/C_{\mathrm{H_2}}}} \cdot \frac{P_{\mathrm{H_2}}}{H_{\mathrm{H_2}}} \qquad (9)$$

wherein, $k_{G,\mathrm{CO}}$ is a gas film mass transfer coefficient of CO in a bubble, in m/s; Pco is a partial pressure of CO in the bubble, in Pa; $H_{\mathrm{co}}$ is a Henry coefficient of CO, in Pa·m³/mol; $C_{i}$, co is a molar concentration of CO in the liquid phase adjacent to the gas-liquid interface, in mol/m³; $C_{i,\mathrm{H_2}}$ is the molar concentration of $H_2$ in the liquid phase adjacent to the gas-liquid interface, in mol/m³; $k_{L,\mathrm{CO}}$ is the liquid side mass transfer coefficient of CO, in m/s; $E_{\mathrm{CO}}$ is the reaction enhancement factor of CO; $r_{\mathrm{Pe}}$ is the macroscopic reaction rate of propylene, in mol/(m³·s); $k_{G,\mathrm{H_2}}$ and $k_{L,\mathrm{H_2}}$ are the gas film mass transfer coefficient and the liquid film mass transfer coefficient of $H_2$, respectively, in m/s; $a$ is a gas-liquid phase interface area, in m²/m³; $E_{\mathrm{H_2}}$ is the reaction enhancement factor of $H_2$; $P_{\mathrm{H_2}}$ is the partial pressure of $H_2$ in a main body of the gas phase in the bubble, in Pa; $H_{\mathrm{H_2}}$ is the Henry coefficient of $H_2$, in Pa·m³/mol;

the mass transfer coefficients in the gas-liquid film of the gas phase component $x$ and $a$ in formulas (7) and (8) have established corresponding structure-effect control mathematical models, which have the following relationship with a system bubble diameter $d_{32}$, respectively;

$$k_{G,x} = \frac{d_{32}}{6 t_{32}} \left[ \frac{D_{Gx} \pi^2 t_{32}}{(d_{32}/2)^2} - \ln \frac{6}{\pi^2} \right] \qquad (10)$$

$$k_{L,x} = 2\sqrt{D_{Lx} v_s / (\pi d_{32})} \qquad (11)$$

$$a = \frac{6 v_G}{d_{32} v_{32}} \qquad (12)$$

wherein, $x$ refers to the gas phase components CO and $H_2$; $D_{Gx}$ is the diffusion coefficient of the gas side of the reactant gas, in m²/s; $D_{Lx}$ is the diffusion coefficient of the liquid side of the reactant gas, in m²/s; $v_G$ is a superficial gas velocity, in m/s;

in formula (10), $t_{32}$ is a residence time of the bubble, in s; if the operating liquid level of the reactor is $H_0$, then:

$$t_{32} = H_0/v_{32} \qquad (13)$$

in formula (11), $v_s$ is a bubble slip velocity, in m/s, which is calculated according to formula (14):

$$v_s = \left| v_{32} - \frac{v_L}{1 - v_G/v_{32}} \right| \qquad (14)$$

in formulas (12) ~ (14), $v_L$ is the superficial liquid velocity, in m/s; $v_{32}$ is a rising velocity of the bubble group, in m/s, and $v_{32}$ is calculated according to formula (15):

$$v_{32} = \frac{(v_0 + v_G + v_L) + \sqrt{(v_0 + v_G + v_L)^2 - 4 v_0 v_G}}{2} \qquad (15)$$

in formula (15), $v_0$ is the rising velocity of a single bubble in an infinite stationary liquid, in m/s, which is calculated according to formula (16):

$$v_0 = \left(\frac{\sigma_L g}{\rho_L}\right)^{1/4} \left[\left(\frac{Mo^{-1/4}}{K_b} d_e^2\right)^{-n} + \left(\frac{2c}{d_e} + \frac{d_e}{2}\right)^{-n/2}\right]^{-1/n} \qquad (16)$$

wherein, $Mo$ in formula (16) is a Morton number, $Mo = g\mu_L^4/(\rho_L \sigma_L^3)$ ; $de$ is a dimensionless bubble diameter, $d_e = d_{32} (\rho_L g/\sigma_L)^{1/2}$ ; $K_b$ is a constant related to physical properties of the system, $K_b = K_{b0} Mo^{-0.038}$, for organic solvents or mixtures, $K_{b0}$=10.2, if $K_b < 12$, then take $K_b$=12 for calculation; constants c and n are also related to the physical properties of the system, and the corresponding empirical values are used according to characteristics of the actual system; $\sigma_L$ is a surface tension of the reaction solution, in N/m; g is an acceleration of gravity, in m/s²; $\rho_L$ is a density of the reaction solution, in kg/m³; $\mu_L$ is a dynamic viscosity of the reaction solution, in Pa s.

Preferably, the step (C) includes:

for the propylene hydro-formylation reaction system, the $Ha$ numbers for reactions of CO and $H_2$ are respectively expressed by formulas (17) and (18):

$$Ha_{CO} = \frac{\sqrt{D_{L,CO}}}{k_{L,CO}} \sqrt{\frac{k_B C_{Pe} C_{H_2} (2 + k_2 x_{Lg})}{1 + a x_{Lg} + b x_{Lg}^2} x_{Rh}} \qquad (17)$$

$$Ha_{H_2} = \frac{\sqrt{D_{L,H_2}}}{k_{L,H_2}} \sqrt{\frac{k_B C_{Pe} C_{CO} (2 + k_2 x_{Lg})}{1 + a x_{Lg} + b x_{Lg}^2} x_{Rh} + k_{Pe} C_{Pe} x_{Rh}} \qquad (18)$$

$E_\infty$ corresponding to the gas component $x$ is expressed by formula (19):

$$E_{\infty,x} = 1 + \frac{D_{L,Pe} C_{Pe}}{D_{L,x} C_{i,x}} \qquad (19)$$

the expression of the reaction enhancement factor $E_x$ corresponding to the gas reaction component $x$ is:

$$E_x = \left( Ha_x \sqrt{\frac{E_\infty - E_x}{E_\infty - 1}} \right) \Big/ \tanh\left( Ha_x \sqrt{\frac{E_\infty - E_x}{E_\infty - 1}} \right) \qquad (20)$$

wherein, $Ha_{CO}$ is a Hatta number of CO; $Ha_{H_2}$ is the Hatta number of $H_2$; $D_{L,CO}$ is the liquid phase diffusion coefficient of CO, in $m^2/s$; $D_{L,H_2}$ is the liquid phase diffusion coefficient of $H_2$, in $m^2/s$; $D_{L,Pe}$ is the diffusion coefficient of propylene in the liquid phase, in $m^2/s$.

Preferably, the step (D) includes:

assuming that the reactor is a fully mixed flow reactor, a material balance calculation of the reaction components propylene, CO and $H_2$ is performed to obtain formulas (21) ~ (23):

$$F_{in,Pe} - Q_L C_{Pe} = R_{Bn} + R_{Bi} + R_{Pe} \qquad (21)$$

$$F_{in,CO} - F_{out,CO} = R_{Bn} + R_{Bi} \qquad (22)$$

$$F_{in,H_2} - F_{out,H_2} = R_{Bn} + R_{Bi} + R_{Pe} \qquad (23)$$

in formulas (21) ~ (23), $F_{in,Pe}$, $F_{in,CO}$ and $F_{in,H_2}$ are respectively molar flow rates of propylene, CO and $H_2$ at a reactor inlet, in mol/s; $Q_L$ is a liquid volume flow at a reactor outlet, in $m^3/s$; and $F_{out,CO}$ and $F_{out,H_2}$ are respectively molar flows of CO and $H_2$ at the reactor outlet, in molls;

assuming that a sum $y_p$ of the partial pressure ratios of the reaction gases $H_2$ and CO and inert gases $N_2$ and methane does not change, formula (24) and formula (25) are obtained according to a relationship between the partial pressures of $H_2$, CO and the inert gases N2 and methane in the gas phase and their molar flow rates:

$$\frac{F_{out,CO}}{F_{out,CO} + F_{out,H_2} + F_I} = \frac{P_{CO}}{y_P P_T} \qquad (24)$$

$$\frac{F_{out,H_2}}{F_{out,CO} + F_{out,H_2} + F_I} = \frac{P_{H_2}}{y_P P_T} \qquad (25)$$

wherein $I$ represents the inert gases N2 and methane etc.; $y_p$ is the ratio of the gas phase partial pressure occupied by the non-liquid phase volatile gas components such as the reaction gas $H_2$, CO, inert gases $N_2$ and methane to the total pressure; $F_I$ is a sum of the molar flows of $I$ at the reactor outlet, in kmol/h; $P_T$ is the reaction operating pressure, in MPa;

the concentrations of propylene, CO and $H_2$ in the liquid phase of the reactor and the molar flows of each component at the reactor outlet are obtained according to the molar flows of propylene, CO and $H_2$ at the reactor inlet, and the formulas (4) ~ (25).

In the evaluation method of the present invention, through construction of the macroscopic kinetic equation of the reaction gas, then through the subsequent establishment of the mathematical expression of the reaction enhancement factor $E_x$ and the establishment of the liquid phase reactant concentration calculation method, the reaction group in the liquid phase, the concentration of the reaction components in the liquid phase and the macroscopic reaction rate are calculated, so as to evaluate the reaction enhancement effect.

The evaluation method of the present invention evaluates the strengthening mass transfer effect of the micro-interface. In actual production, the micro-interface mass transfer enhancement effect is generally achieved by reducing the bubble diameter and increasing the gas-liquid contact area. "Micro" means "micro-nano"; "interface" means that the role of enhancing mass transfer is mainly to improve the gas-liquid contact interface. The evaluation method of the present invention evaluates the strengthening degree of the micro-interface strengthening technology to the reaction system by comparing the parameters such as the macroscopic reaction rate and the mass transfer coefficient of the micro-interface and the macro-interface.

In the step (A), the chemical reaction formula of each reaction in the reaction process is established, and the reaction rate expression of each reaction according to each chemical reaction formula is constructed. In the step (B), based on the reaction formation rate of n-butyraldehyde, isobutyraldehyde and propane obtained in the step (A), and utilizing a condition that the mass transfer rate of CO and $H_2$ in the gas film and the liquid film is equal to the reaction rate in the liquid phase during the reaction, the relationship equation between the macroscopic reaction rate $r_{pe}$ of propane and the bubble scale $d_{32}$ is obtained, and then the mass transfer coefficient of the gas-phase component $x$ in the gas-liquid film and the structure-effect control mathematical model of $a$ are obtained. In the step (C), the mathematical expression of the reaction enhancement factor is established. In the step (D), the material balance of the reaction components propylene, CO and $H_2$ is calculated, and the corresponding expressions are obtained based on the relationship between the partial pressures of all gases and their molar flow rates; by combining the expressions in each step, the concentrations of propylene, CO and $H_2$ in the liquid phase of the reactor, and the molar flow of each component at the outlet can be obtained.

The present invention also provides a micro-interface strengthening reaction system for producing butyraldehyde by propylene hydro-formylation, which is designed by the above calculation method. The reactor designed by the above calculation method can effectively enhance the mass transfer, thereby maximizing the space-time yield rate and realizing the economy of the micro-interface reaction strengthening technology.

Patents with Publication No. CN113061080A, Publication No. CN113072438A, Publication No. CN113061081A and Publication No. CN113041962A have introduced specific micro-interface strengthening reaction systems for propylene hydro-formylation to butyraldehyde, so the present invention will not repeat them. The present invention can calculate the concentration of each reaction component in the liquid phase, thereby judging the strengthening degree of the micro-interface based on the evaluation results, and explaining the strengthening effect of the micro-interface according to the evaluation results.

Compared with the prior art, the beneficial effects of the present invention include:
The present invention aims at realizing the economy of the micro-interface reaction strengthening technology, and improves the space-time yield rate to the greatest extent by determining the evaluation method of the micro-interface reaction strengthening of the oxidation reaction, which is beneficial to optimize the design and operation of the butyraldehyde synthesis reactor.

## BRIEF DESCRIPTION OF THE DRAWINGS

Various other advantages and benefits will become apparent to those of ordinary skill in the art upon reading the following detailed description of the preferred embodiments. The drawings are merely for the purpose of illustrating preferred embodiments and are not to be considered as limiting the invention. Also throughout the drawings, the same reference numerals are used to designate the same components. In the attached drawings:

Fig. 1 is a diagram showing the influence of bubble scale on the gas-liquid phase interface area;

Fig. 2 is a diagram showing the influence of bubble scale on gas side and liquid side volumetric mass transfer coefficients;

Fig. 3 is a diagram showing the influence of bubble scale on the concentration of CO in the liquid phase;

Fig. 4 is a diagram showing the influence of bubble scale on the concentration of $H_2$ in the liquid phase; and

Fig. 5 is a diagram showing the influence of bubble scale on the macroscopic reaction rate of propylene.

## DESCRIPTION OF THE PREFERRED EMBODIMENTS

Exemplary embodiments will be described in detail herein, and examples of which are illustrated in the accompanying drawings. When the following description refers to the accompanying drawings, the same numerals in different drawings refer to the same or similar elements unless otherwise indicated. The implementations described in the following exemplary embodiments do not represent all implementations consistent with the present invention. Rather, they are merely examples of apparatuses and methods consistent with aspects of the present invention are recited in the appended claims.

The terminology used in the present invention is for the purpose of describing particular embodiments only, and is not intended to limit the present invention. As used in the invention and the appended claims, the singular forms "a" and "said" and "the" are intended to include the plural forms as well, unless the context clearly dictates otherwise. It should also be understood that the term "and/or" as used herein refers to and includes any and all possible combinations of one or more of the associated listed items.

It should be understood that although the terms first, second, third, etc. may be used in the present invention to describe various information, the information should not be limited to these terms. These terms are only used to distinguish information of the same type from one another. For example, without departing from the scope of the present invention, first information may also be called second information, and similarly, second information may also be called first information. Depending on the context, the word "if' as used herein may be interpreted as "at" or "when" or "in response to a determination".

In order to illustrate the technical schemes in the present invention more clearly, the following will be described in the form of specific embodiments.

**Embodiments**

This embodiment is based on the evaluation method of the present invention, aiming at the stirred tank reactor and the existing operating conditions of the reaction section of propylene hydro-formylation to produce butyraldehyde in a certain enterprise, to study the effect of the size of bubbles in the reactor on the gas-liquid interface area, gas effect of liquid mass transfer coefficient and macroscopic reaction rate of propylene.

The calculation conditions are as follows:

Reactor operating parameters:

The operating liquid level height of the original reactor $H_0$=9.5 m;

the cross-sectional area of the reactor $S_0$=18.85 m$^2$;

the liquid phase density $\rho_L$=730 kg/m$^3$; the viscosity $\mu_L$=0.195 mPa·s;

the surface tension $\sigma_L$=14.7 mN/m;

the operating pressure $P_T$=1.9 MPa; the operating temperature $T$=362.65 K;

the flow of liquid phase $Q_L$=2.41 × 10$^{-2}$ m$^3$/s;

the molar flow of propylene $F_{Pe}$ =530.13 kmol/h;

the molar flow of CO $F_{CO}$ =400.38 kmol/h;

the molar flow of H$_2$ $F_{H_2}$ =434.32 kmol/h.

An evaluation method for a strengthening degree of a micro-interface reaction of propylene hydro-formylation to produce butyraldehyde in the embodiment of the present invention specifically includes the following steps:

(A) Establishing an expression of a reaction rate of propylene hydro-formylation.

Establishing chemical reaction formulas of all reactions in a process of the micro-interface reaction:

$$CH_3CH = CH_2 + CO + H_2 \rightarrow CH_3CH_2CH_2CHO \qquad (1)$$

$$CH_3CH = CH_2 + CO + H_2 \rightarrow CH_3CH(CH_3)CHO \qquad (2)$$

$$CH_3CH = CH_2 + H_2 \rightarrow CH_3CH_2CH_3 \qquad (3)$$

wherein, formula (1) is a main reaction equation, and formulas (2) ~ (3) are side reaction equations;

constructing reaction rate expressions of all reactions according to the chemical reaction formulas:

$$R_{Bn} = \frac{k_B C_{Pe} C_{H_2} C_{CO} (1 + k_2 x_{Lg})}{1 + a x_{Lg} + x_{Lg}^2} x_{Rh} \qquad (4)$$

$$R_{Bi} = \frac{k_B C_{Pe} C_{H_2} C_{CO}}{1 + a x_{Lg} + b x_{Lg}^2} x_{Rh} \qquad (5)$$

$$R_{pe} = k_{pe} C_{Pe} C_{H_2} x_{Rh} \qquad (6)$$

wherein, $R_{Bh}$, $R_{Bi}$ and $R_{pe}$ are reaction rates of n-butyraldehyde, isobutyraldehyde and propane, respectively, in mol/(m$^3$·s); $k_B$ and $k_{pe}$ are reaction rate constants of butyraldehyde and propane, respectively, in m$^6$/(mol$^2$·s) and m$^3$/(mol·s); $k_2$, $a$ and $b$ are reaction parameters; $C_{Pe}$, $C_{H_2}$ and Cco are concentrations of propylene, H$_2$ and CO in the liquid phase, respectively, in mol/m$^3$; $x_{Lg}$ and $x_{Rh}$ are mass fractions of ligand and catalyst, respectively.

(B) Constructing a macroscopic reaction kinetics equation of a reaction gas.

During the reaction process, mass transfer rates of CO and H$_2$ in a gas film and a liquid film are equal to reaction rates in the liquid phase, and the following formulas are obtained:

$$k_{G,CO} a (P_{CO}/H_{CO} - C_{i,CO}) = k_{L,CO} a E_{CO} (C_{i,CO} - C_{CO}) = R_{Bn} + R_{Bi} \qquad (7)$$

$$k_{G,H_2} a (P_{H_2}/H_{H_2} - C_{i,H_2}) = k_{L,H_2} a E_{H_2} (C_{i,H_2} - C_{H_2}) = R_{Bn} + R_{Bi} + R_{Pe} \qquad (8)$$

the formula (8) is simplified to obtain a macroscopic reaction rate equation of propylene:

$$-r_{Pe} = \frac{1}{\dfrac{1}{k_{G,H_2} a} + \dfrac{1}{k_{L,H_2} a E_{H_2}} + \dfrac{1}{(R_{Bn} + R_{Bi} + R_{Pe})/C_{H_2}}} \cdot \frac{P_{H_2}}{H_{H_2}} \qquad (9)$$

wherein, $k_{G,CO}$ is a gas film mass transfer coefficient of CO in a bubble, in m/s; Pco is a partial pressure of CO in the bubble, in Pa; $H_{co}$ is a Henry coefficient of CO, in Pa·m$^3$/mol; $C_{i}$, co is a molar concentration of CO in the liquid phase adjacent to the gas-liquid interface, in mol/m$^3$; $C_{i,H_2}$ is the molar concentration of H$_2$ in the liquid phase adjacent to the gas-liquid interface, in mol/m$^3$; $k_{L,CO}$ is the liquid side mass transfer coefficient of CO, in m/s; Eco is the reaction enhancement factor of CO; $r_{Pe}$ is the macroscopic reaction rate of propylene, in mol/(m$^3$·s); $k_{G,H_2}$ and $k_{L,H_2}$ are the gas film mass transfer coefficient and the liquid film mass transfer coefficient of H$_2$, respectively, in m/s; $a$ is a gas-liquid phase interface area, in m$^2$/m$^3$; $E_{H_2}$ is the reaction enhancement factor of H$_2$; $P_{H_2}$ is the partial pressure of H$_2$ in a main body of the gas phase in the bubble, in Pa; $H_{H_2}$ is the Henry coefficient of H$_2$, in Pa·m$^3$/mol.

The mass transfer coefficients in the gas-liquid film of the gas phase component $x$ and "$a$" in formulas (7) and (8) have established corresponding structure-effect control mathematical models, which have the following relationship with a system bubble diameter d$_{32}$, respectively;

$$k_{G,x} = \frac{d_{32}}{6 t_{32}} \left[ \frac{D_{Gx} \pi^2 t_{32}}{(d_{32}/2)^2} - \ln \frac{6}{\pi^2} \right] \qquad (10)$$

$$k_{L,x} = 2\sqrt{D_{Lx}v_s/(\pi d_{32})} \qquad\qquad (11)$$

$$a = \frac{6v_G}{d_{32}v_{32}} \qquad\qquad (12)$$

wherein, $x$ refers to the gas phase components CO and $H_2$; $D_{Gx}$ is the diffusion coefficient of the gas side of the reactant gas, in $m^2/s$; $D_{Lx}$ is the diffusion coefficient of the liquid side of the reactant gas, in $m^2/s$; $v_G$ is a superficial gas velocity, in m/s.

In formula (10), $t_{32}$ is a residence time of the bubble, in s; if the operating liquid level of the reactor is $H_0$, then:

$$t_{32} = H_0/v_{32} \qquad\qquad (13).$$

In formula (11), $v_s$ is a bubble slip velocity, in m/s, which is calculated according to formula (14):

$$v_s = \left| v_{32} - \frac{v_L}{1 - v_G/v_{32}} \right| \qquad\qquad (14).$$

In formulas (12) ~ (14), $v_L$ is the superficial liquid velocity, in m/s; $v_{32}$ is a rising velocity of the bubble group, in m/s, and $v_{32}$ is calculated according to formula (15):

$$v_{32} = \frac{(v_0 + v_G + v_L) + \sqrt{(v_0 + v_G + v_L)^2 - 4v_0 v_G}}{2} \qquad\qquad (15).$$

In formula (15), $v_0$ is the rising velocity of a single bubble in an infinite stationary liquid, in m/s, which is calculated according to formula (16):

$$v_0 = \left(\frac{\sigma_L g}{\rho_L}\right)^{1/4} \left[ \left(\frac{Mo^{-1/4}}{K_b} d_e^2\right)^{-n} + \left(\frac{2c}{d_e} + \frac{d_e}{2}\right)^{-n/2} \right]^{-1/n} \qquad\qquad (16)$$

wherein, $Mo$ in formula (16) is a Morton number, $Mo = g\mu_L^4/(\rho_L\sigma_L^3)$; $de$ is a dimensionless bubble diameter, $d_e = d_{32}(\rho_L g/\sigma_L)^{1/2}$; $K_b$ is a constant related to physical properties of the system, $K_b = K_{b0}Mo^{-0.038}$, for organic solvents or mixtures, $K_{b0}=10.2$, if $K_b < 12$, then take $K_b=12$ for calculation; constants c and n are also related to the physical properties of the system, and the corresponding empirical values are used according to characteristics of the actual system; $\sigma_L$ is a surface tension of the reaction solution, in N/m; g is an acceleration of gravity, in $m/s^2$; $\rho_L$ is a density of the reaction solution, in $kg/m^3$; $\mu_L$ is a dynamic viscosity of the reaction solution, in Pa s.

(C) Establishing a mathematical expression of a reaction enhancement factor.

For the propylene hydro-formylation reaction system, the $Ha$ numbers for reactions of CO and $H_2$ are respectively expressed by formulas (17) and (18):

$$Ha_{CO} = \frac{\sqrt{D_{L,CO}}}{k_{L,CO}} \sqrt{\frac{k_B C_{Pe} C_{H_2}(2 + k_2 x_{Lg})}{1 + a x_{Lg} + b x_{Lg}^2} x_{Rh}} \qquad\qquad (17)$$

$$Ha_{H_2} = \frac{\sqrt{D_{L,H_2}}}{k_{L,H_2}} \sqrt{\frac{k_B C_{Pe} C_{CO}(2 + k_2 x_{Lg})}{1 + a x_{Lg} + b x_{Lg}^2} x_{Rh} + k_{Pe} C_{Pe} x_{Rh}} \qquad\qquad (18).$$

$E_\infty$ corresponding to the gas component $x$ is expressed by formula (19):

$$E_{\infty,x} = 1 + \frac{D_{L,\text{Pe}} C_{\text{Pe}}}{D_{L,x} C_{i,x}} \qquad (19).$$

The expression of the reaction enhancement factor $E_x$ corresponding to the gas reaction component $x$ is:

$$E_x = \left( Ha_x \sqrt{\frac{E_\infty - E_x}{E_\infty - 1}} \right) \Big/ \tanh\left( Ha_x \sqrt{\frac{E_\infty - E_x}{E_\infty - 1}} \right) \qquad (20)$$

wherein, $Ha_{\text{CO}}$ is a Hatta number of CO; $Ha_{\text{H}_2}$ is the Hatta number of $H_2$; $D_{L,\text{CO}}$ is the liquid phase diffusion coefficient of CO, in $m^2/s$; $D_{L,\text{H}_2}$ is the liquid phase diffusion coefficient of $H_2$, in $m^2/s$; $D_{L,\text{Pe}}$ is the diffusion coefficient of propylene in the liquid phase, in $m^2/s$.

(D) Calculating a concentration of each reaction component in a liquid phase.

Assuming that the reactor is a fully mixed flow reactor, a material balance calculation of the reaction components propylene, CO and $H_2$ is performed to obtain formulas (21) ~ (23):

$$F_{in,\text{Pe}} - Q_L C_{\text{Pe}} = R_{Bn} + R_{Bi} + R_{\text{Pe}} \qquad (21)$$

$$F_{in,\text{CO}} - F_{out,\text{CO}} = R_{Bn} + R_{Bi} \qquad (22)$$

$$F_{in,\text{H}_2} - F_{out,\text{H}_2} = R_{Bn} + R_{Bi} + R_{\text{Pe}} \qquad (23).$$

In formulas (21) ~ (23), $F_{in,\text{Pe}}$, $F_{in,\text{CO}}$ and $F_{in,\text{H}_2}$ are respectively molar flow rates of propylene, CO and $H_2$ at a reactor inlet, in mol/s; $Q_L$ is a liquid volume flow at a reactor outlet, in $m^3/s$; and $F_{out,\text{CO}}$ and $F_{out,\text{H}_2}$ are respectively molar flows of CO and $H_2$ at the reactor outlet, in molls.

Assuming that a sum $y_p$ of the partial pressure ratios of the reaction gases $H_2$ and CO and inert gases $N_2$ and methane does not change, formula (24) and formula (25) are obtained according to a relationship between the partial pressures of $H_2$, CO and the inert gases N2 and methane in the gas phase and their molar flow rates:

$$\frac{F_{out,\text{CO}}}{F_{out,\text{CO}} + F_{out,\text{H}_2} + F_\text{I}} = \frac{P_{\text{CO}}}{y_P P_T} \qquad (24)$$

$$\frac{F_{out,\text{H}_2}}{F_{out,\text{CO}} + F_{out,\text{H}_2} + F_\text{I}} = \frac{P_{\text{H}_2}}{y_P P_T} \qquad (25)$$

wherein $I$ represents the inert gases N2 and methane etc.; $y_p$ is the ratio of the gas phase partial pressure occupied by the non-liquid phase volatile gas components such as the reaction gas $H_2$, CO, inert gases $N_2$ and methane to the total pressure; $F_I$ is a sum of the molar flows of $I$ at the reactor outlet, in kmol/h; $P_T$ is the reaction operating pressure, in MPa.

The concentrations of propylene, CO and $H_2$ in the liquid phase of the reactor and the molar flows of each component at the reactor outlet are obtained according to the molar flows of propylene, CO and $H_2$ at the reactor inlet, and the formulas (4) ~ (25).

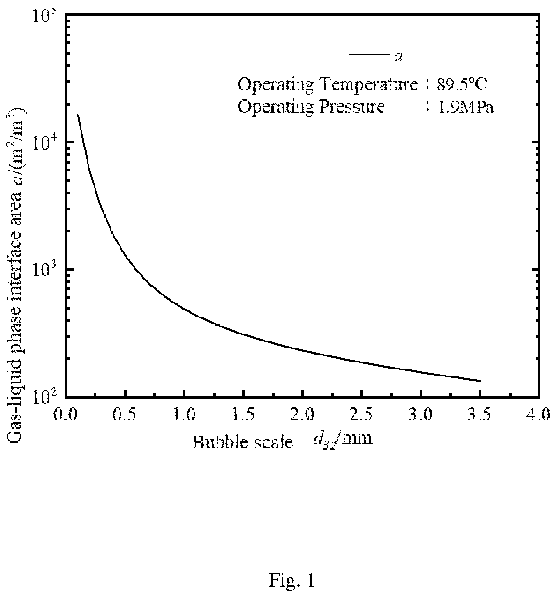

In addition to the relationship between the macroscopic reaction rate and the concentrations of CO and $H_2$ in the liquid phase with the change of the bubble scale, the relationship between the gas-liquid phase interface area and the gas side and liquid side volumetric mass transfer coefficients with the change of the bubble scale can also be obtained. The specific change relationships are shown in Fig. 1 to Fig. 5.

Fig. 1 is a diagram showing the influence of bubble scale $d_{32}$ on the gas-liquid phase interface area $a$. As can be seen from Fig. 1, the gas-liquid phase interface area increases continuously with the decrease of the bubble scale in the reaction system. In this embodiment, the bubble scale is reduced from 3.5 mm to 0.1 mm, and the gas-liquid phase interface area is increased from 134 $m^2/m^3$ to 16425 $m^2/m^3$, which is an increase of about 123 times.

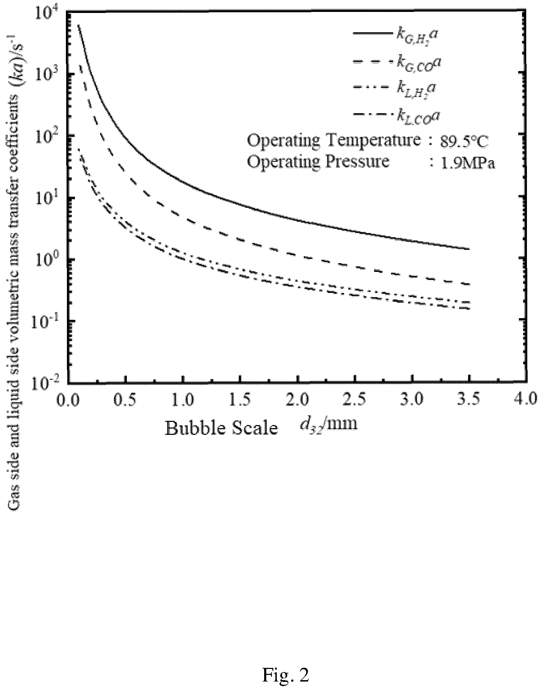

Fig. 2 is a diagram showing the influence of bubble scale $d_{32}$ on gas side and liquid side volumetric mass transfer coefficients. As can be seen from Fig. 2, the gas side and liquid side volumetric mass transfer coefficients of components CO and $H_2$ increase with the decrease of the bubble scale.

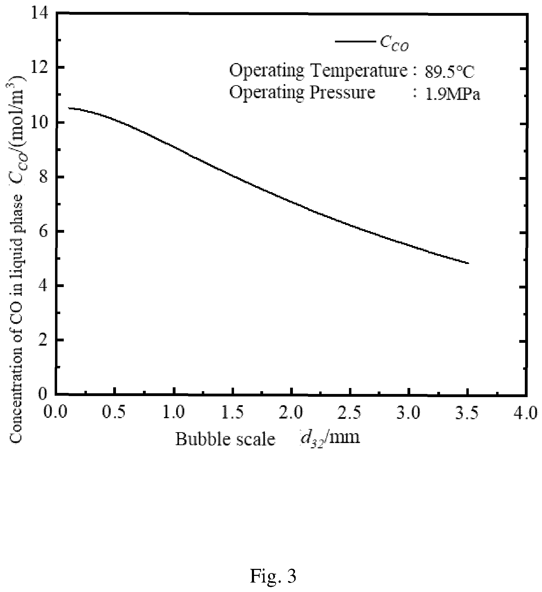

Fig. 3 is a diagram showing the influence of bubble scale $d_{32}$ on the concentration of CO in the liquid phase. As can be seen from Fig. 3, the smaller the bubble scale, the greater the concentration of CO in the liquid phase. In this embodiment, the bubble scale decreased from 3.5 mm to 0.1 mm, and the concentration of CO in the liquid phase increased from 4.9 mol/m$^3$ to 10.5 mol/m$^3$, which is an increase of about 116.4%.

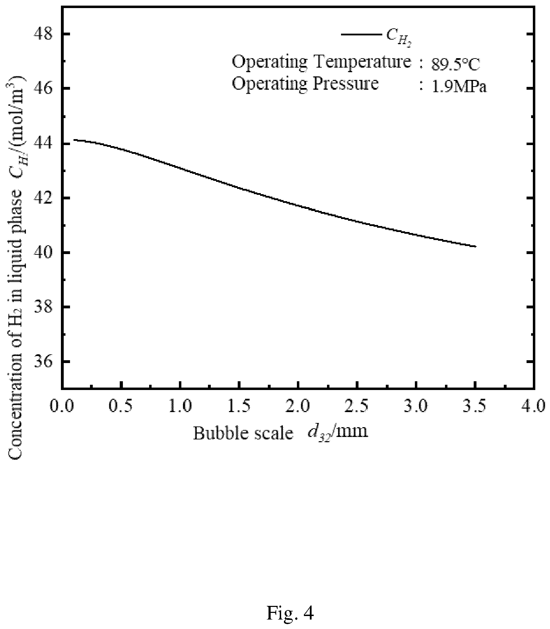

Fig. 4 is a diagram showing the influence of bubble scale $d_{32}$ on the concentration of $H_2$ in the liquid phase. As can be seen from Fig. 4, the smaller the bubble scale, the greater the concentration of $H_2$ in the liquid phase. In this embodiment, the bubble scale decreased from 3.5 mm to 0.1 mm, and the concentration of $H_2$ in the liquid phase increased from 40.2 mol/m$^3$ to 44.1 mol/m$^3$, which is an increase of about 9.7%.

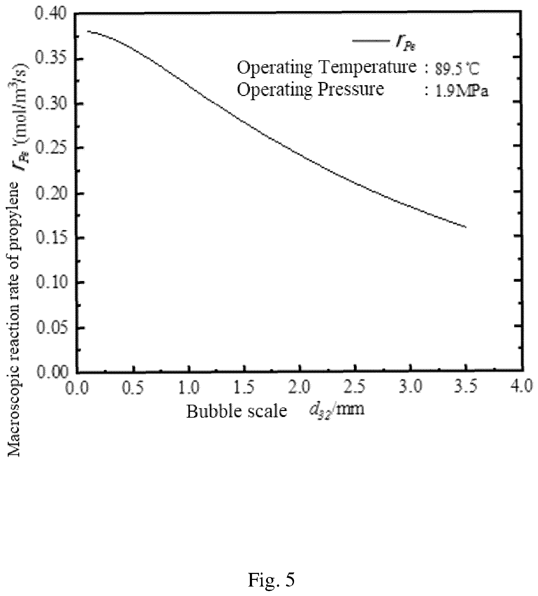

Fig. 5 is a diagram showing the influence of bubble scale $d_{32}$ on the macroscopic reaction rate of propylene. As can be seen from Fig. 5, the macroscopic reaction rate of propylene increases with the decrease of the bubble scale. In this embodiment, the bubble scale decreased from 3.5 mm to 0.1 mm, and the macroscopic reaction rate of propylene increased from 0.160 mol/(m$^3$•s) to 0.381 mol/(m$^3$·s), which is an increase of about 137.5%.

As can be seen from Fig. 1 to Fig. 5, when the bubble scale $d_{32}$ decreases, the gas-liquid phase interface area increases continuously, the concentrations of CO and $H_2$ in the liquid phase increase, the gas side and liquid side volumetric mass transfer coefficients increase, and the macroscopic reaction rate of propylene also increases. It can be seen that the reduction of bubble scale is beneficial to improve the reaction efficiency of propylene hydro-formylation to produce butyraldehyde.

Even if the above parameters are not clearly listed in the calculation formulas, they can be obtained through simple calculations based on the obtained result parameters.

The above descriptions are merely preferred embodiments of the present invention, and are not intended to limit the present invention. Any modifications, equivalent replacements, improvements, etc. made within the spirit and principles of the present invention shall be included in the present invention within the scope of protection.

**Claims**

1. An evaluation method for a strengthening degree of a micro-interface reaction of propylene hydro-formylation to produce butyraldehyde, **characterized in that**, comprising the following steps:

    (A) establishing an expression of a reaction rate of propylene hydro-formylation;
    (B) constructing a macroscopic reaction kinetics equation of a reaction gas;
    (C) establishing a mathematical expression of a reaction enhancement factor; and
    (D) calculating a concentration of each reaction component in a liquid phase.

2. The evaluation method according to claim 1, wherein the step (A) comprises:

    establishing chemical reaction formulas of all reactions in a process of the micro-interface reaction:

$$\mathbf{CH_3CH = CH_2 + CO + H_2 \rightarrow CH_3CH_2CH_2CHO} \qquad (1)$$

$$\mathbf{CH_3CH = CH_2 + CO + H_2 \rightarrow CH_3CH(CH_3)CHO} \qquad (2)$$

$$\mathbf{CH_3CH = CH_2 + H_2 \rightarrow CH_3CH_2CH_3} \qquad (3)$$

    wherein, formula (1) is a main reaction equation, and formulas (2) ~ (3) are side reaction equations;
    constructing reaction rate expressions of all reactions according to the chemical reaction formulas:

$$R_{Bn} = \frac{k_B C_{Pe} C_{H_2} C_{CO}(1 + k_2 x_{Lg})}{1 + a x_{Lg} + x_{Lg}{}^2} x_{Rh} \qquad (4)$$

$$R_{Bi} = \frac{k_B C_{Pe} C_{H_2} C_{CO}}{1 + a x_{Lg} + b x_{Lg}{}^2} x_{Rh} \qquad (5)$$

$$R_{pe} = k_{pe} C_{Pe} C_{H_2} x_{Rh} \qquad (6)$$

wherein, $R_{Bh}$, $R_{Bi}$ and $R_{pe}$ are reaction rates of n-butyraldehyde, isobutyraldehyde and propane, respectively, in mol/(m³·s); $k_B$ and $k_{pe}$ are reaction rate constants of butyraldehyde and propane, respectively, in m⁶/(mol²·s) and m³/(mol·s); $k_2$, $a$ and $b$ are reaction parameters; $C_{Pe}$, $C_{H_2}$ and Cco are concentrations of propylene, $H_2$ and CO in the liquid phase, respectively, in mol/m³; $x_{Lg}$ and $x_{Rh}$ are mass fractions of ligand and catalyst, respectively.

3. The evaluation method according to claim 1, wherein the step (B) comprises:

during the reaction process, mass transfer rates of CO and $H_2$ in a gas film and a liquid film are equal to reaction rates in the liquid phase, and the following formulas are obtained:

$$k_{G,CO} a (P_{CO}/H_{CO} - C_{i,CO}) = k_{L,CO} a E_{CO} (C_{i,CO} - C_{CO}) = R_{Bn} + R_{Bi} \qquad (7)$$

$$k_{G,H_2} a (P_{H_2}/H_{H_2} - C_{i,H_2}) = k_{L,H_2} a E_{H_2} (C_{i,H_2} - C_{H_2}) = R_{Bn} + R_{Bi} + R_{Pe} \qquad (8)$$

the formula (8) is simplified to obtain a macroscopic reaction rate equation of propylene:

$$-r_{Pe} = \frac{1}{\dfrac{1}{k_{G,H_2} a} + \dfrac{1}{k_{L,H_2} a E_{H_2}} + \dfrac{1}{(R_{Bn} + R_{Bi} + R_{Pe})/C_{H_2}}} \cdot \frac{P_{H_2}}{H_{H_2}} \qquad (9)$$

wherein, $k_{G,CO}$ is a gas film mass transfer coefficient of CO in a bubble, in m/s; $P_{CO}$ is a partial pressure of CO in the bubble, in Pa; $H_{co}$ is a Henry coefficient of CO, in Pa·m³/mol; $C_i$, co is a molar concentration of CO in the liquid phase adjacent to the gas-liquid interface, in mol/m³; $C_{i,H2}$ is the molar concentration of $H_2$ in the liquid phase adjacent to the gas-liquid interface, in mol/m³; $k_{L,CO}$ is the liquid side mass transfer coefficient of CO, in m/s; $E_{CO}$ is the reaction enhancement factor of CO; $r_{Pe}$ is the macroscopic reaction rate of propylene, in mol/(m³·s); $k_{G,H2}$ and $k_{L,H2}$ are the gas film mass transfer coefficient and the liquid film mass transfer coefficient of $H_2$, respectively, in m/s; $a$ is a gas-liquid phase interface area, in m²/m³; $E_{H_2}$ is the reaction enhancement factor of $H_2$; $P_{H2}$ is the partial pressure of $H_2$ in a main body of the gas phase in the bubble, in Pa; $H_{H_2}$ is the Henry coefficient of $H_2$, in Pa·m³/mol;

the mass transfer coefficients in the gas-liquid film of the gas phase component $x$ and "$a$" in formulas (7) and (8) have established corresponding structure-effect control mathematical models, which have the following relationship with a system bubble diameter $d_{32}$, respectively;

$$k_{G,x} = \frac{d_{32}}{6 t_{32}} \left[ \frac{D_{Gx} \pi^2 t_{32}}{(d_{32}/2)^2} - \ln \frac{6}{\pi^2} \right] \qquad (10)$$

$$k_{L,x} = 2\sqrt{D_{Lx} v_s / (\pi d_{32})} \qquad (11)$$

$$a = \frac{6 v_G}{d_{32} v_{32}} \qquad (12)$$

wherein, $x$ refers to the gas phase components CO and $H_2$; $D_{Gx}$ is the diffusion coefficient of the gas side of the reactant gas, in $m^2/s$; $D_{Lx}$ is the diffusion coefficient of the liquid side of the reactant gas, in $m^2/s$; $v_G$ is a superficial gas velocity, in m/s;

in formula (10), $t_{32}$ is a residence time of the bubble, in s; if the operating liquid level of the reactor is $H_0$, then:

$$t_{32} = H_0/v_{32} \qquad (13)$$

in formula (11), $v_s$ is a bubble slip velocity, in m/s, which is calculated according to formula (14):

$$v_s = \left| v_{32} - \frac{v_L}{1 - v_G/v_{32}} \right| \qquad (14)$$

in formulas (12) ~ (14), $v_L$ is the superficial liquid velocity, in m/s; $v_{32}$ is a rising velocity of the bubble group, in m/s, and $v_{32}$ is calculated according to formula (15):

$$v_{32} = \frac{(v_0 + v_G + v_L) + \sqrt{(v_0 + v_G + v_L)^2 - 4v_0 v_G}}{2} \qquad (15)$$

in formula (15), $v_0$ is the rising velocity of a single bubble in an infinite stationary liquid, in m/s, which is calculated according to formula (16):

$$v_0 = \left( \frac{\sigma_L g}{\rho_L} \right)^{1/4} \left[ \left( \frac{Mo^{-1/4}}{K_b} d_e^2 \right)^{-n} + \left( \frac{2c}{d_e} + \frac{d_e}{2} \right)^{-n/2} \right]^{-1/n} \qquad (16)$$

wherein, $Mo$ in formula (16) is a Morton number, $Mo = g\mu_L^4/(\rho_L\sigma_L^3)$; $de$ is a dimensionless bubble diameter, $d_e = d_{32}(\rho_L g/\sigma_L)^{1/2}$; $K_b$ is a constant related to physical properties of the system, $K_b = K_{b0}Mo^{-0.038}$, for organic solvents or mixtures, $K_{b0}$=10.2, if $K_b$ < 12, then take $K_b$=12 for calculation; constants c and n are also related to the physical properties of the system, and the corresponding empirical values are used according to characteristics of the actual system; $\sigma_L$ is a surface tension of the reaction solution, in N/m; $g$ is an acceleration of gravity, in $m/s^2$; $\rho_L$ is a density of the reaction solution, in $kg/m^3$; $\mu_L$ is a dynamic viscosity of the reaction solution, in Pa s.

4.   The evaluation method according to claim 3, wherein the step (C) comprises:

for the propylene hydro-formylation reaction system, the $Ha$ numbers for reactions of CO and $H_2$ are respectively expressed by formulas (17) and (18):

$$Ha_{CO} = \frac{\sqrt{D_{L,CO}}}{k_{L,CO}} \sqrt{\frac{k_B C_{Pe} C_{H_2} (2 + k_2 x_{Lg})}{1 + a x_{Lg} + b x_{Lg}^2} x_{Rh}} \qquad (17)$$

$$Ha_{H_2} = \frac{\sqrt{D_{L,H_2}}}{k_{L,H_2}} \sqrt{\frac{k_B C_{Pe} C_{CO} (2 + k_2 x_{Lg})}{1 + a x_{Lg} + b x_{Lg}^2} x_{Rh} + k_{Pe} C_{Pe} x_{Rh}} \qquad (18)$$

$E_\infty$, corresponding to the gas component $x$ is expressed by formula (19):

$$E_{\infty,x} = 1 + \frac{D_{L,\text{Pe}} C_{\text{Pe}}}{D_{L,x} C_{i,x}} \qquad (19)$$

the expression of the reaction enhancement factor $E_x$ corresponding to the gas reaction component $x$ is:

$$E_x = \left( Ha_x \sqrt{\frac{E_\infty - E_x}{E_\infty - 1}} \right) \Big/ \tanh\left( Ha_x \sqrt{\frac{E_\infty - E_x}{E_\infty - 1}} \right) \qquad (20)$$

wherein, $Ha_{\text{CO}}$ is a Hatta number of CO; $Ha_{\text{H2}}$ is the Hatta number of $H_2$; $D_{L,\text{CO}}$ is the liquid phase diffusion coefficient of CO, in m$^2$/s; $D_{L,\text{H}_2}$ is the liquid phase diffusion coefficient of $H_2$, in m$^2$/s; $D_{L,\text{Pe}}$ is the diffusion coefficient of propylene in the liquid phase, in m$^2$/s.

5. The evaluation method according to claim 1, wherein the step (D) comprises:

assuming that the reactor is a fully mixed flow reactor, a material balance calculation of the reaction components propylene, CO and $H_2$ is performed to obtain formulas (21) ~ (23):

$$F_{in,\text{Pe}} - Q_L C_{\text{Pe}} = R_{Bn} + R_{Bi} + R_{\text{Pe}} \qquad (21)$$

$$F_{in,\text{CO}} - F_{out,\text{CO}} = R_{Bn} + R_{Bi} \qquad (22)$$

$$F_{in,\text{H}_2} - F_{out,\text{H}_2} = R_{Bn} + R_{Bi} + R_{\text{Pe}} \qquad (23)$$

in formulas (21) ~ (23), $F_{in,Pe}$, $F_{in,CO}$ and $F_{in,H2}$ are respectively molar flow rates of propylene, CO and $H_2$ at a reactor inlet, in mol/s; $Q_L$ is a liquid volume flow at a reactor outlet, in m$^3$/s; and $F_{out,CO}$ and $F_{out,H2}$ are respectively molar flows of CO and $H_2$ at the reactor outlet, in molls;

assuming that a sum $y_p$ of the partial pressure ratios of the reaction gases $H_2$ and CO and inert gases $N_2$ and methane does not change, formula (24) and formula (25) are obtained according to a relationship between the partial pressures of $H_2$, CO and the inert gases N2 and methane in the gas phase and their molar flow rates:

$$\frac{F_{out,\text{CO}}}{F_{out,\text{CO}} + F_{out,\text{H}_2} + F_\text{I}} = \frac{P_{\text{CO}}}{y_P P_T} \qquad (24)$$

$$\frac{F_{out,\text{H}_2}}{F_{out,\text{CO}} + F_{out,\text{H}_2} + F_\text{I}} = \frac{P_{\text{H}_2}}{y_P P_T} \qquad (25)$$

wherein $I$ represents the inert gases N2 and methane etc.; $y_p$ is the ratio of the gas phase partial pressure occupied by the non-liquid phase volatile gas components such as the reaction gas $H_2$, CO, inert gases $N_2$ and methane to the total pressure; $F_I$ is a sum of the molar flows of $I$ at the reactor outlet, in kmol/h; $P_T$ is the reaction operating pressure, in MPa;

the concentrations of propylene, CO and $H_2$ in the liquid phase of the reactor and the molar flows of each component at the reactor outlet are obtained according to the molar flows of propylene, CO and $H_2$ at the reactor inlet, and the formulas (4) ~ (25).

6. A micro-interface strengthening reaction system for producing butyraldehyde by propylene hydro-formylation, is designed and obtained by adopting the evaluation method according to claim 1.

Fig. 1

Fig. 2

Fig. 3

Fig. 4

Fig. 5

<div style="text-align:center">**INTERNATIONAL SEARCH REPORT**</div>

| International application No. |
|---|
| **PCT/CN2022/103299** |

**A. CLASSIFICATION OF SUBJECT MATTER**

G16C 20/10(2019.01)i;  G16C 10/00(2019.01)i

According to International Patent Classification (IPC) or to both national classification and IPC

**B. FIELDS SEARCHED**

Minimum documentation searched (classification system followed by classification symbols)

G16C

Documentation searched other than minimum documentation to the extent that such documents are included in the fields searched

Electronic data base consulted during the international search (name of data base and, where practicable, search terms used)

CNTXT, DWPI, WPABS, WPABSC, ENTXTC, 中国期刊网全文数据库 CNKI, STN: 南京延长反应技术研究院有限公司, 南京大学, 张志炳, 周政, 微气泡, 微界面, 气泡, 破碎, 粉碎, 微米, 速率, 浓度, 丙烯, 烯烃, 氢甲酰化, 羰基化, 强化, olefin?, propylene, hydroformyla+, carbonyla+, bubble?, micro, interfac+, rate, concentrat+

**C. DOCUMENTS CONSIDERED TO BE RELEVANT**

| Category* | Citation of document, with indication, where appropriate, of the relevant passages | Relevant to claim No. |
|---|---|---|
| PX | CN 113971988 A (NANJING YANCHANG REACTION TECHNOLOGY RESEARCH INSTITUTE CO., LTD.) 25 January 2022 (2022-01-25)<br>    claims 1-6 | 1-6 |
| PY | CN 114019107 A (NANJING YANCHANG REACTION TECHNOLOGY RESEARCH INSTITUTE CO., LTD.) 08 February 2022 (2022-02-08)<br>    description, paragraphs 5-57 | 1, 6 |
| Y | CN 113061081 A (NANJING YANCHANG REACTION TECHNOLOGY RESEARCH INSTITUTE CO., LTD.) 02 July 2021 (2021-07-02)<br>    description, paragraph 12 | 1, 6 |
| Y | CN 113075091 A (NANJING YANCHANG REACTION TECHNOLOGY RESEARCH INSTITUTE CO., LTD.) 06 July 2021 (2021-07-06)<br>    description, paragraphs 6-69 | 1, 6 |
| Y | CN 113061080 A (NANJING YANCHANG REACTION TECHNOLOGY RESEARCH INSTITUTE CO., LTD.) 02 July 2021 (2021-07-02)<br>    description, paragraph 4 | 1, 6 |

☑ Further documents are listed in the continuation of Box C.      ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **15 September 2022** | **28 September 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2022/103299** |

**C.    DOCUMENTS CONSIDERED TO BE RELEVANT**

| Category* | Citation of document, with indication, where appropriate, of the relevant passages | Relevant to claim No. |
|---|---|---|
| Y | CN 111302917 A (CHINA NAT OFFSHORE OIL CORP. et al.) 19 June 2020 (2020-06-19) description, paragraphs [0009]-[0045] | 1, 6 |
| A | CN 102826967 A (CHINA PETROLEUM & CHEMICAL CORP. et al.) 19 December 2012 (2012-12-19) entire document | 1-6 |
| A | US 4528404 A (EXXON RESEARCH AND ENGINEERING CO.) 09 July 1985 (1985-07-09) entire document | 1-6 |

Form PCT/ISA/210 (second sheet) (January 2015)

**INTERNATIONAL SEARCH REPORT**
Information on patent family members

International application No.

**PCT/CN2022/103299**

| Patent document cited in search report | | | Publication date (day/month/year) | Patent family member(s) | | | Publication date (day/month/year) |
|---|---|---|---|---|---|---|---|
| CN | 113971988 | A | 25 January 2022 | None | | | |
| CN | 114019107 | A | 08 February 2022 | None | | | |
| CN | 113061081 | A | 02 July 2021 | None | | | |
| CN | 113075091 | A | 06 July 2021 | None | | | |
| CN | 113061080 | A | 02 July 2021 | None | | | |
| CN | 111302917 | A | 19 June 2020 | None | | | |
| CN | 102826967 | A | 19 December 2012 | CN | 102826967 | B | 22 July 2015 |
| US | 4528404 | A | 09 July 1985 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107563051 A
- CN 113061080 A
- CN 113072438 A
- CN 113061081 A
- CN 113041962 A